# EUROPEAN PATENT APPLICATION

(11) **EP 2 998 742 A1**
(43) Date of publication of application: **23.03.2016**
(21) Application number: 14797564.3
(22) Date of filing: 16.05.2014
(51) Int. Cl.: G01N 33/574, A61K 31/7072, A61P 35/00, G01N 33/48, C07K 16/18

(54) **THERAPEUTIC EFFECT PREDICTION METHOD FOR COLORECTAL CANCER PATIENT IN WHOM EXPRESSION OF TK1 PROTEIN HAS INCREASED**

(30) Priority: 17.05.2013 JP 2013105082
(71) Applicant: Taiho Pharmaceutical Co., Ltd., Chiyoda-ku, Tokyo 101-8444 (JP)
(72) Inventor: SAKAMOTO, Kazuki, Tsukuba-shi Ibaraki 300-2611 (JP); ITO, Masanobu, Tokyo 101-8444 (JP)
(74) Representative: Hoffmann Eitle
(86) International application number: PCT/JP2014/063085
(87) International publication number: WO 2014/185528

(57) **Abstract**

The present invention provides a method for predicting the therapeutic effect of chemotherapy using an antitumor agent containing trifluridine and tipiracil hydrochloride at a molar ratio of 1:0.5 on a colorectal cancer patient, the method comprising the following steps (1) to (3). As the means for solving the problem, the following invention is provided: a method comprising (1) detecting the expression of TK1 protein by an immunohistochemical method in tumor cells contained in a biological sample obtained from the patient; (2) based on the detection results obtained in step (1), classifying the tumor cells into positive cells and negative cells, and calculating the percentage of positive cells in the tumor cells; and (3) based on the calculation results obtained in step (2), predicting that when the percentage is 30% or more, the patient is likely to sufficiently respond to chemotherapy using the antitumor agent containing trifluridine and tipiracil hydrochloride at a molar ratio of 1:0.5.

## Description

### Technical Field

### Cross-Reference to Related Application

This application claims priority based on Japanese Patent Application No. 2013-105082 filed on May 17, 2013, the entire contents of which are incorporated herein by reference.

The present invention relates to a method for predicting the therapeutic effect of chemotherapy using an antitumor agent containing trifluridine (hereinafter also referred to as "FTD") and tipiracil hydrochloride (hereinafter also referred to as "TPI") at a molar ratio of 1:0.5, and also relates to an antitumor agent and a kit.

### Background Art

The standard therapy for treating colorectal cancer patients has been performed, typically, with chemotherapy using fluoropyrimidine-based antitumor agents (e.g., a combination of 5-fluorouracil (5-FU) and leucovorin (LV)), and optionally, with multidrug chemotherapy (FOLFIRI, FOLFOX, or the like) that additionally uses irinotecan or oxaliplatin. Such methods have achieved a certain therapeutic effect (NPL 1). However, when a colorectal cancer patient becomes refractory or intolerant to these standard therapies using 5-FU, irinotecan, or oxaliplatin, the choice of antitumor agent that can significantly prolong their survival is very limited.

Meanwhile, a combination drug containing FTD and TPI at a molar ratio of 1:0.5 (hereinafter also referred to as "FTD-TPI combination drug") is under development as a new antitumor agent against colorectal cancer. The FTD-TPI combination drug is an antitumor agent containing FTD, which is an active ingredient having an antitumor effect, and TPI, which is a component suppressing the degradation of FTD by thymidine phosphorylase (TP), at a molar ratio of 1:0.5. This agent is known to exhibit antitumor effects due to the inhibition of thymidylate synthase (TS) by FTD and the incorporation of FTD phosphorylated by thymidine kinase 1 (TK1) into DNA (NPL 2). Further, since large-scale clinical tests targeted at colorectal cancer patients refractory or intolerant to standard therapy have confirmed excellent extension of overall survival by the FTD-TPI combination drug, the drug is highly anticipated as an antitumor agent against colorectal cancer (NPL 3).

Moreover, due to the action mechanism of the FTD-TPI combination drug, TK1, etc., are expected to serve as factors for predicting the therapeutic effect of the FTD-TPI combination drug. However, analysis of the expression of TK1 protein by an H-score method, which is an immunohistochemical evaluation method, in a clinical test of the FTD-TPI combination drug targeted at colorectal cancer patients who are refractory or intolerant to standard therapy reported that there was no correlation between the expression of TK1 protein and overall survival (NPL 4). The "H-score method" is an evaluation method based on the H-score determined by the following calculation formula: H-score = ∑(staining intensity x percentage of positive cells (%)) (staining intensity 0: unstained; staining intensity 1: weak staining; staining intensity 2: moderate staining; staining intensity 3: strong staining) (J Clin Pathol. 1995; 48: 876-878).

As explained above, despite the vigorous development of chemotherapies for colorectal cancer patients, their therapeutic effects are still insufficient. In particular, effective chemotherapies have not substantially been established for colorectal cancer patients who are refractory or intolerant to standard therapy.

### Citation List

### Non-patent Literature

NPL 1: NCCN Clinical Practice Guidelines in Oncology (NCCN Guidelines™) ; Colon Cancer (Version 3, 2011), Rectal Cancer (Version 4, 2011)
NPL 2: Int J Mol Med. 2004; 13(2): 249-55.
NPL 3: Eur J Cancer. 2011; 47 (Suppl.1): 392, #6005.
NPL 4: Eur J Cancer. 2011; 47 (Suppl.1): 421, #6099.

### Summary of Invention

### Technical Problem

An object of the present invention is to provide chemotherapy for colorectal cancer patients, the chemotherapy ensuring a significant survival-prolongation effect and having fewer side effects.

### Solution to Problem

Under the circumstances wherein a correlation between the expression of TK1 protein and the therapeutic effect of the FTD-TPI combination drug was denied, as stated above, the present inventors focused on the relationship between the percentage of positive cells (in particular, cells evaluated as a score of 2+ or 3+ by an immunohistochemical method) in all tumor cells in a biological sample and the therapeutic effect of the FTD-TPI combination drug, and found that the FTD-TPI combination drug was more likely to have a significant effect on colorectal cancer patients in whom the percentage of positive cells was 30% or more, compared with patients in whom the percentage was less than 30%. Thus, the present invention has been accomplished.

Cancer patients with a high expression of TK1 are generally known to have a poor prognosis. The remarkably high therapeutic effect of the FTD-TPI combination drug when the percentage of positive cells is 30% or more is an unexpected result for a person skilled in the art.

Specifically, the present invention includes the following methods, antitumor agents, and kit.

Item 1. A method for predicting a therapeutic effect of chemotherapy using an antitumor agent containing trifluridine and tipiracil hydrochloride at a molar ratio of 1:0.5 on a colorectal cancer patient, the method comprising the following steps (1) to (3) :
(1) detecting the expression of TK1 protein by an immunohistochemical method in tumor cells contained in a biological sample obtained from the patient;
(2) based on the detection results obtained in step (1), classifying the tumor cells into positive cells and negative cells, and calculating the percentage of positive cells in the tumor cells; and
(3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.

Item 2. The method according to Item 1, wherein the positive cells are evaluated as 2+ or 3+ based on staining intensity.

Item 3. The method according to Item 1 or 2, wherein the colorectal cancer patient is refractory or intolerant to standard therapy.

Item 4. An antitumor agent containing trifluridine and tipiracil hydrochloride at a molar ratio of 1:0.5 for a colorectal cancer patient, wherein the patient is predicted to be likely to sufficiently respond to chemotherapy using the antitumor agent, by a method comprising the following steps (1) to (3):
(1) detecting the expression of TK1 protein by an immunohistochemical method in tumor cells contained in a biological sample obtained from the patient;
(2) based on the detection results obtained in step (1), classifying the tumor cells into positive cells and negative cells, and calculating the percentage of positive cells in the tumor cells; and
(3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.

Item 5. A kit for predicting a therapeutic effect of chemotherapy using an antitumor agent containing trifluridine and tipiracil hydrochloride at a molar ratio of 1:0.5 on a colorectal cancer patient, the kit comprising an antibody that specifically recognizes TK1 protein as a reagent, wherein the therapeutic effect is predicted by the following steps (1) to (3):
(1) detecting the expression of TK1 protein by an immunohistochemical staining method in tumor cells contained in a biological sample obtained from the patient;
(2) based on the detection results obtained in step (1), classifying the tumor cells into positive cells and negative cells, and calculating the percentage of positive cells in the tumor cells; and
(3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.

Item 6. A method for treating a colorectal cancer patient comprising the following steps (1) to (4):
(1) detecting the expression of TK1 protein by an immunohistochemical staining method in tumor cells contained in a biological sample obtained from the patient;
(2) based on the detection results obtained in step (1), classifying the tumor cells into positive cells and negative cells, and calculating the percentage of positive cells in the tumor cells;
(3) based on the calculation results obtained in step (2), predicting that when the percentage is 30% or more, the patient is likely to sufficiently respond to chemotherapy using an antitumor agent containing trifluridine and tipiracil hydrochloride at a molar ratio of 1:0.5; and
(4) administering the antitumor agent to the patient who is predicted to be likely to sufficiently respond to the chemotherapy in step (3).

Item 7. An antitumor agent containing trifluridine and tipiracil hydrochloride at a molar ratio of 1:0.5, wherein the antitumor agent is for use in the treatment of a colorectal cancer patient who is predicted to be likely to sufficiently respond to chemotherapy using the antitumor agent, by a method comprising the following steps (1) to (3):
(1) detecting the expression of TK1 protein by an immunohistochemical method in tumor cells contained in a biological sample obtained from the patient;
(2) based on the detection results obtained in step (1), classifying the tumor cells into positive cells and negative cells, and calculating the percentage of positive cells in the tumor cells; and
(3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.

The present invention further includes the following embodiments.

- A method for testing a therapeutic effect of chemotherapy using an antitumor agent containing trifluridine and tipiracil hydrochloride at a molar ratio of 1:0.5 on a colorectal cancer patient, the method comprising the following steps (1) to (3):
   (1) detecting the expression of TK1 protein by an immunohistochemical method in tumor cells contained in a biological sample obtained from the patient;
   (2) based on the detection results obtained in step (1), classifying the tumor cells into positive cells and negative cells, and calculating the percentage of positive cells in the tumor cells; and
   (3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.

- A method for examining whether chemotherapy using an antitumor agent containing trifluridine and tipiracil hydrochloride at a molar ratio of 1:0.5 exhibits a therapeutic effect on a colorectal cancer patient, the method comprising the following steps (1) to (3):
   (1) detecting the expression of TK1 protein by an immunohistochemical method in tumor cells contained in a biological sample obtained from the patient;
   (2) based on the detection results obtained in step (1), classifying the tumor cells into positive cells and negative cells, and calculating the percentage of positive cells in the tumor cells; and
   (3) comparing the calculation results obtained in step (2) with a standard that when the percentage is 30% or more, the patient is likely to sufficiently respond to the chemotherapy using the antitumor agent containing trifluridine and tipiracil hydrochloride at a molar ratio of 1:0.5.

### Advantageous Effects of Invention

The prediction method of the present invention can provide chemotherapy that ensures more significant survival-prolongation effects for colorectal cancer patients (in particular, colorectal cancer patients refractory or intolerant to standard therapy who were less responsive to antitumor agents and had few choices of antitumor agents for significantly prolonging their survival).

### Description of Embodiments

The prediction method of the present invention is to predict, test, or examine whether chemotherapy using an antitumor agent containing FTD and TPI at a molar ratio of 1:0.5 exhibits a sufficient therapeutic effect on a colorectal cancer patient, based on the percentage of TK1 positive cells in tumor cells.

TK1 protein, which is used as an index in the present invention, is an enzyme that synthesizes deoxythymidine monophosphate by phosphorylation of thymidine, and is known to be involved in the synthesis of pyrimidine, which is essential for DNA synthesis. Moreover, high expression of TK1 protein is reportedly associated with tumor malignancy in many types of cancer.

The target patients of the present invention are colorectal cancer patients. In the present invention, "colorectal cancer" refers to a malignant tumor generated in the colon or rectum, including primary colorectal cancer, locally recurrent colorectal cancer, and colorectal cancer that has spread to other tissues (e.g., the liver). The "colorectal cancer patients" include not only patients currently having colorectal cancer tumor tissue, but also patients who have undergone resection of colorectal cancer tumor tissue. Therefore, in this specification, the therapeutic effect of chemotherapy encompasses shrinkage of colorectal cancer, suppression of proliferation, prolongation of patient survival, as well as suppression of the recurrence of colorectal cancer after the resection of tumor tissue.

Further, the treatment history of the colorectal cancer patients of the present invention is not particularly limited insofar as the patients can endure administration of the above antitumor agent; however, the target patients are preferably colorectal cancer patients who are refractory or intolerant to standard therapy, in terms of the prediction accuracy of the present invention. In the present invention, "standard therapy" refers to chemotherapy that is standardly used for the treatment of colorectal cancer, and that uses a fluoropyrimidine-based antitumor agent (e.g., 5-fluorouracil (5-FU)), irinotecan, or oxaliplatin. Specific examples thereof include chemotherapy that uses a fluoropyrimidine-based antitumor agent (e.g., a combination of 5-fluorouracil (5-FU) and leucovorin (LV)), and combination chemotherapy (FOLFIRI, FOLFOX, etc.) that uses irinotecan or oxaliplatin, in addition to the fluoropyrimidine-based antitumor agent. Herein, the condition "refractory or intolerant to standard therapy" refers to a state in which the patient is not responsive to the standard therapy (including the cases where progression (PD) is observed during the standard therapy, the cases where cancer recurrence is found during or within 6 months after the standard therapy conducted as postoperative adjuvant chemotherapy, and the like), a state in which the patient is unable to withstand the administration of a standard amount of the antitumor agent due to aggravation of disease or side effects, or the like. The condition "refractory or intolerant to standard therapy" can also be expressed as "difficult to conduct standard therapy."

The antitumor agent of the present invention contains trifluridine and tipiracil hydrochloride at a molar ratio of 1:0.5, and is known to exhibit antitumor effects mainly on solid cancer, such as colorectal cancer, by oral administration (WO96/30346).

"Trifluridine" (synonym: α,α,α-trifluorothymidine) is a known nucleic acid derivative in which the methyl group at the 5 position of thymidine is replaced by a trifluoromethyl group, and is known to have an antitumor effect by its DNA synthesis inhibiting action (J. Am. Chem. Soc. 84: 3597-3598, 1962; J. Med. Chem., 7: 1-5, 1964; Biochemistry, 33: 15086-15094, 1994). Trifluridine is represented by the following chemical formula (1) :

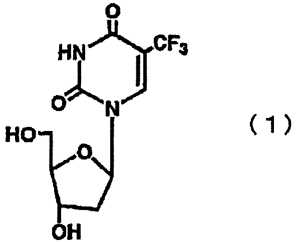

"Tipiracil hydrochloride" (chemical name: 5-chloro-6-[(2-iminopyrrolidin-1-yl)methyl]pyrimidine-2,4(1H,3H)-dione hydrochloride) is a known compound having a thymidine phosphorylase activity-inhibiting action, and is known to have the following effects: enhancement of antitumor effects (WO96/30346), inhibition of cancer metastasis (WO98/13045), relief of gastrointestinal disorders caused by antitumor agents (WO00/56337), and potentiation of radiation therapy (WO2008001502). Tipiracil hydrochloride is represented by the following chemical formula (2):

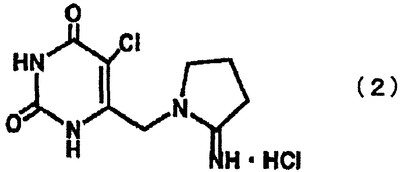

The antitumor agent of the present invention may be provided as a combination drug (preparation containing a plurality of active ingredients) by formulating trifluridine and tipiracil hydrochloride into a single dosage form (single-formulation type); or may be provided as single active ingredient preparations by formulating the active ingredients into a plurality of dosage forms (multiple-formulation type). Of these, preferable is a combination drug of trifluridine and tipiracil hydrochloride.

The dosage form of the antitumor agents is not particularly limited, and can be suitably selected depending on the purpose of the treatment. Specific examples thereof include oral preparations (such as tablets, coated tablets, powders, granules, capsules, and fluids), injections, suppositories, patches, and ointments. Of these, the combination drug containing trifluridine and tipiracil hydrochloride is preferably in the form of an oral preparation. Each antitumor agent can be prepared by a commonly known method, using one or more pharmacologically acceptable carriers in accordance with each dosage form. Examples of the carriers include those that are widely used in common drugs, such as excipients, binders, disintegrators, lubricants, diluents, solubilizing agents, suspending agents, tonicity adjusting agents, pH adjusters, buffers, stabilizers, colorants, sweetening agents, and flavoring agents.

The "chemotherapy using an antitumor agent containing trifluridine and tipiracil hydrochloride at a molar ratio of 1:0.5" in the present invention refers to chemotherapy in which at least the antitumor agent of the present invention is administered; this includes chemotherapy using the FTD-TPI combination drug alone, and chemotherapy using the antitumor agent of the present invention and other antitumor agents in combination.

The administration schedule of the chemotherapy is suitably selected according to conditions, such as the patient's age, sex, stage of disease, presence or absence of metastasis, and history of treatment. For example, it is preferable to repeat the following 4-week administration course. In each course, the antitumor agent of the present invention is administered from Day 1 to Day 5, and from Day 8 to Day 12, 2 to 4 times a day in an FTD amount of 20 to 80 mg/m² (per body surface area)/day, preferably 2 to 3 times a day in an FTD amount of 50 to 70 mg/m² (per body surface area)/day, more preferably 2 times a day in an FTD amount of 70 mg/m² (per body surface area)/day. The frequency of administration per day is not particularly limited, and is 2 to 4 times, preferably 2 or 3 times, more preferably 2 times.

The chemotherapy of the present invention may be preoperative adjuvant chemotherapy in which the chemotherapy is performed before resection of a tumor, or postoperative adjuvant chemotherapy in which the chemotherapy is performed after resection of a tumor.

The production and sales of an antitumor agent containing FTD and TPI at a molar ratio of 1:0.5 have been approved in Japan under the name "Lonsurf combination tablet."

In the present invention, "therapeutic effect" can be evaluated based on the tumor-shrinking effect, the effect of suppressing recurrence, the effect of prolonging survival, etc., as described above. The effect of suppressing recurrence can be represented by the extension of recurrence-free survival or the degree of improvement in recurrence rate. The effect of prolonging survival can be represented by the degree of extension of the median of overall survival or progression-free survival. "Sufficiently respond to chemotherapy using an antitumor agent containing trifluridine and tipiracil hydrochloride at a molar ratio of 1:0.5" means a superior therapeutic effect by the administration of the antitumor agent of the present invention, including a significant extension of survival, and a significant suppression of recurrence, compared with a treatment without the administration of the antitumor agent of the present invention.

The prediction method of the present invention comprises steps (1) to (3) described below.

Step (1) is a step of detecting the expression of TK1 protein by an immunohistochemical method in tumor cells contained in a biological sample obtained from a colorectal cancer patient.

The biological sample is not particularly limited, as long as it is obtained from a colorectal cancer patient and contains tumor cells. Examples thereof include body fluid (such as blood and urine), tissue, extracts thereof, and cultures of obtained tissue; tissue containing tumor cells is preferable. The method for obtaining the biological sample can be suitably selected according to the type of biological sample.

The method for detecting the expression of TK1 protein in the present invention is not particularly limited, as long as it is an immunohistochemical method that allows semi-quantitative assessment of the expression of TK1 protein for each tumor cell as the evaluation score based on staining intensity. A known detection method can be used. The evaluation score based on staining intensity in the present invention is preferably a two-stage evaluation including 0 (unstained) and 1+ (staining), or a four-stage evaluation including 0 (unstained), 1+ (weak staining), 2+ (moderate staining), and 3+ (strong staining). The four-stage evaluation can be performed, for example, according to the four-stage evaluation of the expression intensity of HER2 protein in Histopathology 2008; 52: 797-805.

The biological sample is prepared as tissue section samples from biopsied tissues or organs, by being subjected to a process appropriate for the detection method. Moreover, tissue section samples can be, for example, fresh, frozen, or formalin-, alcohol-, acetone- or otherwise fixed and/or paraffin-embedded and deparaffinized. Preferably, one of the serial sections of formalin-fixed paraffin-embedded blocks is stained with hematoxylin and eosin (HE) in advance, cancer cells are distinguished from normal cells based on cell form, and only tumor cell portions are used for the assessment of the present invention. For negative or positive controls for staining intensity, formalin-fixed paraffin-embedded cell lines (several types of lines whose protein expression levels are known in advance) may be employed. When there are no control specimens, a plurality of specimens are assessed simultaneously to confirm the overall distribution of staining intensity of the specimens, and then staining intensity may be set. Regarding assessed sites, it is desirable to assess the entire specimen; however, it is possible to assess only one representative field.

The antibody specifically recognizing TK1 protein used for detection may be a monoclonal antibody or a polyclonal antibody, or may be an antibody fragment, such as a Fab fragment or a F(ab')2 fragment. The host species of the antibody is not particularly limited.

Moreover, this antibody can generally be produced by a known method based on the amino acid sequence information (GenBank ID: AAH07986) of known human TK1 (e.g., Current protocols in Molecular Biology edit. Ausubel et al. (1987), Publish. John Wiley and Sons. Section 11.12-11.13). For example, when the antibody of the present invention is a polyclonal antibody, it can be obtained by immunizing a test animal with the aforementioned polypeptide expressed in *E. coli* and purified by an ordinary method, or a polypeptide synthesized so as to have a partial amino acid sequence of the aforementioned polypeptide by an ordinary method; and obtaining the antibody from the serum of the immunized animal by an ordinary method. In contrast, for example, when the antibody of the present invention is a monoclonal antibody, it can be obtained by immunizing a test animal with the aforementioned polynucleotide expressed in *E*. *coli,* etc., and purified by an ordinary method, or a polypeptide synthesized so as to have a partial amino acid sequence of the aforementioned polynucleotide by an ordinary method; fusing the spleen cell obtained from the test animal and a myeloma cell to synthesize a hybridoma cell; and obtaining the antibody from the hybridoma cell. Alternatively, a known anti-human TK1 antibody may be used (Hybridoma. 2001; 20(1): 25-34).

Step (2) is a step of classifying the assessed tumor cells into positive cells and negative cells based on the detection results in step (1), and calculating the percentage of positive cells in the tumor cells.

The positive cells in the present invention refer to tumor cells in which the expression of TK1 protein is confirmed by an immunohistochemical method. Specifically, the positive cells refer to tumor cells that are evaluated as 1+ in the case of a two-stage evaluation, or 2+ or 3+ in the case of a four-stage evaluation, based on the staining intensity for each tumor cell obtained in step (1).

Step (3) is a step of predicting that when the percentage of positive cells in the tumor cells is 30% or more, the patient is likely to sufficiently respond to the chemotherapy using the antitumor agent of the present invention, based on the calculation results obtained in step (2). In the present invention, the threshold (cut-off point) of the percentage of positive cells used as the diagnostic criteria is 30%. Analysis performed using various cut-off points, including Examples provided later, showed that the stratification effect was highest when the cut-off point was 30%, in terms of prediction accuracy and therapeutic effect (extension of overall survival, etc.).

Thus, the therapeutic effect of chemotherapy using the antitumor agent of the present invention on a colorectal cancer patient can be predicted.

The present invention also provides a therapeutic method that exhibits excellent therapeutic effects of chemotherapy, such as shrinkage of colorectal cancer, suppression of proliferation, prolongation of patient survival, as well as suppression of the recurrence of colorectal cancer after the resection of tumor tissue, by administering the antitumor agent of the present invention to a patient who is predicted to be likely to sufficiently respond to chemotherapy using the antitumor agent, by the prediction method of the present invention. The present invention provides such a therapeutic method and usage form of the antitumor agent of the present invention.

The present invention also provides a kit for predicting the therapeutic effect of chemotherapy using the antitumor agent of the present invention on a colorectal cancer patient. The kit of the present invention is suitably used to predict the therapeutic effect of chemotherapy by the prediction method of the present invention, described above.

The kit of the present invention is characterized in that it comprises an antibody specifically recognizing TK1 protein as a reagent. The kit of the present invention may contain other reagents and/or equipment for enforcing an immunohistochemical method, such as means for detecting the antibody specifically recognizing TK1 protein (i.e., primary antibody), negative or positive controls for staining intensity, and the like. For example, a second antibody can be used as the means for detecting the primary antibody. The second antibody may be labeled, if necessary, with an enzyme, such as peroxidase or alkaline phosphatase; a fluorescent or luminescent substance, such as fluorescein isothiocyanate (FITC) or carboxymethylindocyanine (Cy3); or the like. When the second antibody is labeled with an enzyme, the kit of the present invention preferably further contains a chromogenic substrate, such as 3,3'-diaminobenzidine (DAB) or 3,3',5,5'-tetramethylbenzidine (TMB). The kit of the present invention may further contain instructions for use, manuals, or the like that describe procedures for enforcing the prediction method of the present invention.

### Examples

Examples are given below to illustrate the present invention in more detail. Needless to say, the present invention is not limited to these Examples.

### Example 1

Progressive recurring colorectal cancer patients (169 cases) who were refractory or intolerant to standard therapy including 5-FU, irinotecan, and oxaliplatin, and who had a treatment history of at least 2 regimens, were divided into a group (112 cases) administered with an antitumor agent containing FTD and TPI at a molar ratio of 1:0.5 (hereinafter referred to as the "FTD-TPI combination drug administration group") and a placebo group (57 cases). There was no significant background difference between these two groups (including percentage of male patients (FTD-TPI combination drug administration group: 57.1%; placebo group: 49.1%), average age (FTD-TPI combination drug administration group: 63; placebo group: 62), ECOG PS (Eastern Cooperative Oncology Group Performance Status) 0 (FTD-TPI combination drug administration group: 64.3%; placebo group: 61.4%), and percentage of the patients having a treatment history of 3 or more regimens (FTD-TPI combination drug administration group: 84.8%; placebo group: 77.2%)). In the FTD-TPI combination drug administration group, during the 4-week administration course, the FTD-TPI combination drug was administered twice a day in an FTD amount of 35 mg/m² (per body surface area)/dose, that is, 70 mg/m² (per body surface area)/day in total per day, from Day 1 to Day 5 and from Day 8 to Day 12. This administration schedule was regarded as one course, and the course was repeatedly performed. On the other hand, no antitumor agent, including the FTD-TPI combination drug, was given to the placebo group.

The overall survival (OS) was evaluated in all cases. Further, lesional tissues were obtained from 145 cases (FTD-TPI combination drug administration group: 95 cases, placebo group: 50 cases) out of all cases as biological samples before medication. After formalin fixation, paraffin-embedding treatment was performed, and the samples were sliced into 3- to 4-µm sections. After the sliced samples were dried in a paraffin melter overnight, the samples were deparaffinized in xylene and ethanol. Further, endogenous peroxidase was blocked by 0.3% hydrogen peroxide/methanol treatment, and the antigen was activated by pressure cooker treatment (for 10 minutes) in 1 mM EDTA (pH 8.0). After washing with running water and washing with PBS (-), the primary antibody (anti-TK1 rabbit polyclonal antibody recognizing the amino acid sequence at the C terminal of human TK1 protein represented by SEQ ID NO: 1 (FKKASGQPAGPDNKENC); produced by Taiho Pharmaceutical Co., Ltd.) was reacted at ordinary temperature overnight. Further, after washing with PBS (-), a peroxidase-second antibody-bound polymer reagent (Simple Stain MAX-PO (R), produced by Nichirei Biosciences Inc.) was reacted at room temperature for 30 minutes, followed by washing with PBS (-). After the color was developed using a Simple Stain DAB solution (produced by Nichirei Biosciences Inc.), the core was stained with Carrazzi's Hematoxylin solution, followed by a dehydrating and permeating operation, and the samples were mounted with Malinol. Thus, TK1-stained samples were prepared.

Next, the expression of TK1 protein in each tumor cell was evaluated under a microscope separately by two pathology experts depending on the staining intensity of the cytoplasm of the tumor cells using a four-stage evaluation including 0 (unstained), 1+ (weak staining), 2+ (moderate staining), and 3+ (strong staining). The percentage of tumor cells evaluated as 2+ or 3+ in all tumor cells was calculated for each case.

Table 1 shows the number of cases in each group when 20% or 30% was used as the cut-off point for classifying the cases into a high TK1 expression group (High) and a low TK1 expression group (Low). Further, Table 2 shows the median overall survival and hazard ratio (HR) of the high and low expression groups at each cut-off point. P values for evaluating significant differences between the high and low expression groups were calculated by the log-rank test.

When the cut-off point was 20%, the median overall survival of the high TK1 expression group of the placebo group was 5.9 months, whereas the median overall survival of the high TK1 expression group of the FTD-TPI combination drug administration group was 7.8 months. There was a small difference in the hazard ratio between the FTD-TPI combination drug administration group and the placebo group. In contrast, when the cut-off point was 30%, the median overall survival of the high TK1 expression group of the placebo group was 7.7 months, whereas the median overall survival of the high TK1 expression group of the FTD-TPI combination drug administration group was 10.4 months. There was a significant difference in the hazard ratio between the FTD-TPI combination drug administration group and the placebo group.

**Table 1**

| | **TK1 Cutoff=20%** | | **TK1 Cutoff=30%** | | **Total** |
|---|---|---|---|---|---|
| | **High** | **Low** | **High** | **Low** | |
| **FTD·TPI combination drug** | **43** | **56** | **27** | **72** | **99** |
| **Placebo** | **21** | **30** | **13** | **38** | **51** |
| **Total** | **64** | **86** | **40** | **110** | **150** |

**Table 2**

| | **TK1 Cutoff=20%** | | **TK1 Cutoff=30%** | |
|---|---|---|---|---|
| | **High** | **Low** | **High** | **Low** |
| **FTD·TPI combination drug (N=99)** | **7.8 vs. 9.0** | | **10.4 vs. 7.7** | |
| | **HR=0.98** | | **HR=0.51** | |
| | **95%Cl [0.57,1.68] (p=0.93)** | | **95%Cl [0.27, 0.97] (p=0.04)** | |
| **Placebo (N=51)** | **5.9 vs. 6.9** | | **4.9 vs. 7.2** | |
| | **HR=1.16** | | **HR=1.56** | |
| | **95%Cl [0.59, 2.31] (p=0.66)** | | **95%Cl [0.63, 3.89] (p=0.34)** | |

The above results demonstrated that although the FTD-TPI combination drug was clinically useful for colorectal cancer patients regardless of the expression of TK1 protein, the FTD-TPI combination drug particularly showed a significantly excellent therapeutic effect on patients in which the percentage of tumor cells evaluated as 2+ or 3+ according to the expression of TK1 protein in all tumor cells was 30% or more. Cancer patients with a high expression of TK1 are generally known to have a bad prognosis, and this therapeutic effect is an unexpected result for a person skilled in the art.

## Claims

1. A method for predicting a therapeutic effect of chemotherapy using an antitumor agent containing trifluridine and tipiracil hydrochloride at a molar ratio of 1:0.5 on a colorectal cancer patient, the method comprising the following steps (1) to (3) :
(1) detecting the expression of TK1 protein by an immunohistochemical method in tumor cells contained in a biological sample obtained from the patient;
(2) based on the detection results obtained in step (1), classifying the tumor cells into positive cells and negative cells, and calculating the percentage of positive cells in the tumor cells; and
(3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.

2. The method according to claim 1, wherein the positive cells are evaluated as 2+ or 3+ based on staining intensity.

3. The method according to claim 1 or 2, wherein the colorectal cancer patient is refractory or intolerant to standard therapy.

4. An antitumor agent containing trifluridine and tipiracil hydrochloride at a molar ratio of 1:0.5 for a colorectal cancer patient, wherein the patient is predicted to be likely to sufficiently respond to chemotherapy using the antitumor agent, by a method comprising the following steps (1) to (3):
(1) detecting the expression of TK1 protein by an immunohistochemical method in tumor cells contained in a biological sample obtained from the patient;
(2) based on the detection results obtained in step (1), classifying the tumor cells into positive cells and negative cells, and calculating the percentage of positive cells in the tumor cells; and
(3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.

5. A kit for predicting a therapeutic effect of chemotherapy using an antitumor agent containing trifluridine and tipiracil hydrochloride at a molar ratio of 1:0.5 on a colorectal cancer patient, the kit comprising an antibody that specifically recognizes TK1 protein as a reagent, wherein the therapeutic effect is predicted by the following steps (1) to (3):
(1) detecting the expression of TK1 protein by an immunohistochemical staining method in tumor cells contained in a biological sample obtained from the patient;
(2) based on the detection results obtained in step (1), classifying the tumor cells into positive cells and negative cells, and calculating the percentage of positive cells in the tumor cells; and
(3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.

6. A method for treating a colorectal cancer patient comprising the following steps (1) to (4):
(1) detecting the expression of TK1 protein by an immunohistochemical staining method in tumor cells contained in a biological sample obtained from the patient;
(2) based on the detection results obtained in step (1), classifying the tumor cells into positive cells and negative cells, and calculating the percentage of positive cells in the tumor cells;
(3) based on the calculation results obtained in step (2), predicting that when the percentage is 30% or more, the patient is likely to sufficiently respond to chemotherapy using an antitumor agent containing trifluridine and tipiracil hydrochloride at a molar ratio of 1:0.5; and
(4) administering the antitumor agent to the patient who is predicted to be likely to sufficiently respond to the chemotherapy in step (3).

7. An antitumor agent containing trifluridine and tipiracil hydrochloride at a molar ratio of 1:0.5, wherein the antitumor agent is for use in the treatment of a colorectal cancer patient who is predicted to be likely to sufficiently respond to chemotherapy using the antitumor agent, by a method comprising the following steps (1) to (3):
(1) detecting the expression of TK1 protein by an immunohistochemical method in tumor cells contained in a biological sample obtained from the patient;
(2) based on the detection results obtained in step (1), classifying the tumor cells into positive cells and negative cells, and calculating the percentage of positive cells in the tumor cells; and
(3) based on the calculation results obtained in step (2), predicting that when the percentage is 30% or more, the patient is likely to sufficiently respond to the chemotherapy using the antitumor agent containing trifluridine and tipiracil hydrochloride at a molar ratio of 1:0.5.


### SEQUENCE LISTING

<110> TAIHO PHARMACEUTICAL CO., LTD.
<120> Method for predicting therapy effect in colorectal cancer patients with enhanced TK1 protein expression
<130> P14-064
<150> JP 2013-105082
   <151> 2013-05-17
<160> 1
<170> PatentIn version 3.4
<210> 1
   <211> 17
   <212> PRT
   <213> Artificial Sequence
<220>
   <223> Synthetic peptide
<400> 1
   Phe Lys Lys Ala Ser Gly Gln Pro Ala Gly Pro Asp Asn Lys Glu Asn
   1               5                   10                  15
   Cys